# EUROPEAN PATENT APPLICATION

(11) **EP 3 766 573 A1**
(43) Date of publication of application: **20.01.2021**
(21) Application number: 19726137.3
(22) Date of filing: 15.03.2019
(51) Int. Cl.: B01J 20/289, B01J 20/32, B01D 15/38, C12N 15/10

(54) **CHROMATOGRAPHIC MATRIX, METHOD AND USES THEREOF**

(30) Priority: 15.03.2018 PT 2018110626
(71) Applicant: Universidade de Aveiro, 3810-193 Aveiro (PT); Universidade Da Beira Interior, 6201-001 Covilhã (PT)
(72) Inventor: FREIRE MARTINS, Mara Guadalupe, 3810-696 Aveiro (PT); CARVALHO NEVES, Márcia, 3830-154 Ílhavo (PT); QUARESMA HENRIQUES PEDRO, Augusto, 3850-089 Albergaria-a-velha (PT); PEREIRA DE SOUSA, Fani, 6200-026 Covilhã (PT); DE SAMPAIO RODRIGUES QUEIROZ, João António, 6300-748 Guarda (PT); CLAUDINO MARTINS, João Carlos, 6120-017 Envendos (PT); NUNES PEREIRA, Patrícia Alexandra, 3525-308 Canas De Senhorim (PT)
(74) Representative: Patentree
(86) International application number: PCT/IB2019/052133
(87) International publication number: WO 2019/175849

(57) **Abstract**

The present disclosure concerns the development of a novel stationary phase for the separation of nucleic acids in the context of preparative chromatography, using a chromatographic matrix, preferably a macroporous matrix, particularly a matrix of an epoxylated methacrylic polymer modified with the ionic liquid (IL) 1-methylimidazolium chloride as multimodal ligand.

## Description

### Technical domain

The present disclosure concerns the development of a novel stationary phase for the separation of nucleic acids [ribonucleic acid genomic deoxyribonucleic acid (gDNA), preferably wherein the RNA fraction comprises low molecular weight RNA (ImwRNA) and ribosomal RNA (rRNA)] in the context of preparative chromatography, using a chromatographic matrix, preferably a macroporous matrix, particularly a matrix of an epoxylated methacrylic polymer, modified with 1-methylimidazole after binding of the (3-chloropropyl)trimethoxysilane as spacer arm. As a result of the immobilization process, the ionic liquid (IL) 1-methylimidazolium chloride is formed which acts as multimodal ligand. In particular, IL 1-methylimidazolium chloride presents in its structure an aromatic ring which can establish hydrophobic interactions, and additionally, also presents a positive charge center with the counter-ion chloride allowing anionic exchange with negatively charged species. As a result, IL 1-methylimidazolium chloride is defined as a multimodal ligand. The characterization of the chromatographic matrix by solid-state cross-polarization magic angle spinning Nuclear Magnetic Resonance (NMR) with spectrum acquired with ¹³C (CPMAS) and elemental analysis, as well as the determination of the point of zero charge (PZC) proved the correct functionalization of the stationary phase with the IL. Concerning its chromatographic behavior, the separation of ImwRNA and gDNA, or the separation of ImwRNA, rRNA and gDNA, present in a bacterial lysate was successfully attained in conditions that mainly promote electrostatic interactions, presenting this matrix a high dynamic binding capacity, robustness, specificity and reproducibility. In general, this novel chromatographic matrix presents a wide applicability, not only for obtaining RNA for therapeutic purposes, but also for diagnostic purposes, or for applications in molecular biology.

### State of the art

Nucleotides show peculiar biological and chemical characteristics, playing therefore important roles in distinct areas of biochemistry, biology and medicine. Throughout several decades, RNA was thought to act as a simple intermediate between DNA and proteins, not having therefore aroused great interest among researchers. However, this scenario has been changing once research intensification on RNA functions has led it to be recognized as a regulator of a wide set of biological processes, making it currently an important target in both basic and applied research [1]. On the other hand, RNA is becoming to play an important role in the (bio)pharmaceutical industry, due to its high potential as biopharmaceuticals for the treatment of several human diseases [1].

Currently, studies conducted in order to determine the structure and function of RNA, or the establishment of RNA-based therapies usually require high quantities of intact, pure and biologically active target RNA [2]. Indeed, independently of the method used for RNA preparation and its final application, usually there is a need to use RNA separation methods, considering the removal of contaminants. In particular, for the production of RNA with potential pharmaceutical application, this has to be obtained according to good manufacturing practices (GMP), being it necessary that the separation method allows the RNA to comply with the requirements required by regulatory agencies, namely the Food and Drug Administration (FDA), the European Medicines Agency (EMA) and the World Health Organization (WHO) [1]. The most common methods used for RNA purification include denaturing polyacrylamide gel electrophoresis, and different chromatographic strategies such as ion pairing high performance liquid chromatography, reversed-phase or molecular exclusion [2]. In general, these methods present diverse drawbacks, namely, they are relatively expensive, very laborious methods, the RNA is recovered with purity levels distant from those desired and partially denatured due to the experimental conditions used [1, 2]. Affinity chromatography with immobilized amino acids as ligands, including arginine histidine has allowed to overcome some of these obstacles [2]. Ideally, the most suitable chromatographic support for RNA purification and separation should present diverse characteristics, namely, high selectivity, specificity and binding capacity, high mechanical and chemical stability and be available at a low cost [2]. Matrices for preparative chromatographic of nucleic acids have been developed by immobilizing ligands in diverse supports including agarose, dextran, methacrylates, polystyrene, silica, among others [1]. These are commercially available in a pre-packed form or are packed in the laboratory in glass or polypropylene columns by the researchers. On the other hand, monolithic-based chromatographic strategies for the separation of RNA were also recently reported.

The state of the art in the purification of RNA for the most diverse applications encompasses, as described, the use of chromatographic matrices, which can be regenerated and reused several times, virtually for samples obtained via *in vitro* transcription, chemical synthesis, or recombinantly produced. In parallel, and resorting to packaging of anion-exchange disposable chromatographic columns, several kits are also commercially available for the isolation of genomic DNA from bacteria, namely: "Bacterial genomic miniprep kit®" (Sigma-aldrich, St Louis, MO, United States of America), "PureLink® Microbiome DNA Purification kit" (ThermoFischer Scientific, Waltham, MA, United States of America), "Wizard® Genomic DNA Purification Kit" (Promega, Madison, United States of America), "Genomic-tip 20/G®" (Qiagen, Hilden, Germany), "Quick-DNA™ Microprep Plus Kit®" (Zymo Research, Irvine, United States of America), among others. On the other hand, there are also commercial kits for the isolation of total RNA from bacteria: "Total RNA bacteria Purification kit®" (Sigma-aldrich, St Louis, MO, United States of America), "PureLink® RNA Mini kit" (ThermoFischer Scientific, Waltham, MA, United States of America), "SV Total RNA Isolation System®" (Promega, Madison, United States of America), "RNeasy® Mini Kit" (Qiagen, Hilden, Germany), "Quick-RNA® Fungal/Bacterial RNA Microprep" (Zymo Research, Irvine, United States of America), among others. However, at least the "Quick-RNA® Fungal/Bacterial RNA Microprep" (Zymo Research, Irvine, United States of America) does not allow the separation of ribosomal RNA from other bacterial RNAs.

The tendency on the development of novel strategies for the separation of RNA has been the implementation and/or development of novel, more selective, robust and reproductible chromatographic ligands [1]. In this way, emerge the ILs, salts which are present in the liquid state at low temperatures - below 100 °C -, which are exclusively composed by ions, specifically large and asymmetric organic cations, combined with organic or inorganic anions [3, 4]. In general, these compounds are non-flammable and display a negligible volatility, which makes them an excellent alternative to conventional organic solvents, allowing to decrease the environmental footprint and the cost of different processes. Besides that, they present high chemical, electrochemical and thermal stabilities, reinforcing the idea that these compounds are usually considered safe and benign for the environment [4].

ILs are generally described as designer solvents once due to the number of possible combinations of their ions, their properties can be optimized and adjusted, allowing the synthesis of compounds that fit within different specific applications [3]. Indeed, envisaging the replacement of volatile and toxic solvents, they have been used for dissolving a wide range of materials [4]. The diverse benefic characteristics of ILs led them to be also applied in chromatographic processes, foreseeing to improve, virtually, any separation.

Specifically, ILs can be used in liquid chromatography in different ways [4], namely: as additives in mobile phases, as ligands in stationary phases, or acting themselves as stationary phases in chromatography.

In particular, distinct immobilization patterns have been used for the development of stationary phases containing ILs, namely: immobilization or co-immobilization of the IL through its cations or anions [5]. These stationary phases have been mostly obtained by chromatographic matrices composed by silica, or monoliths, in which ILs are immobilized.

A common feature to the previously reported works is that they were applied in the field of analytical chromatography, i.e., were based on the separation and/or quantification of the different analytes. On the other hand, there are no documents reporting the use of stationary phases with immobilized ILs as ligands for the separation of different classes of nucleic acids from the same bacterial extract.

Up to date, different methods involving ILs were patented for the synthesis of polynucleotides [US20050106685], solubilization and preservation of nucleic acids [EP1736542 A1], to increase the sensitivity and amplification efficiency of nucleic acids from biological materials [US9598726 B2], or for the purification of nucleic acids [US8101744 B2]. The invention [US8101744 B2] comprises the isolation and purification of nucleic acids (herring sperm DNA, calf thymus DNA, or RNA isolated from baker's yeast) involving the adsorption of the nucleic acid to a solid support (composed by silica gel, glass fibers, quartz fibers, and celite, or mineral substrates from the group comprising metal oxides, or mixed metal oxides such as alumina, titanium, or zirconium) in the presence of an aqueous solution of an IL, wherein it facilitates the binding of the nucleic acid to the solid support.

### Summary

The present disclosure aims to present a novel chromatographic matrix based on the functionalization of a novel macroporous support with the IL 1-methylimidazolium chloride (MP-SilPrMImCI), using (3-chloropropyl)trimethoxysilane as the spacer arm, envisaging its application in the field of preparative chromatography, i.e. in the separation of ImwRNA from gDNA, or in the separation of ImwRNA, rRNA and gDNA with high efficiency, specificity, robustness and selectivity.

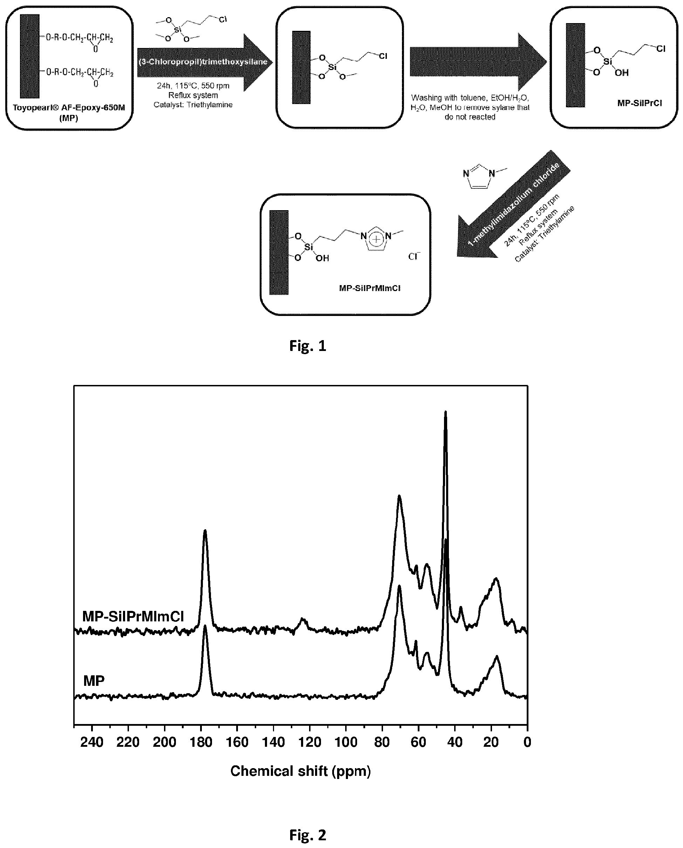

The preparation of the chromatographic matrix MP-SilPrMImCl encompassed two main stages, according to **Fig. 1**: 1) Functionalization of the chromatographic matrix, preferably a macroporous matrix, particularly a matrix of an epoxylated methacrylic polymer, commercially obtained (Toyopearl® AF-Epoxy-650M, Tosoh Biosciences, GmbH, Greisheim, Germany) with (3-chloropropyl)trimethoxysilane (MP-SilPrCl), followed by 2) Functionalization of the chromatographic matrix, preferably a macroporous matrix, particularly a matrix of an epoxylated methacrylic polymer functionalized with (3-chloropropyl)trimethoxysilane obtained in 1), with 1-methylimidazole, leading to the formation of the IL 1-methylimidazolium chloride which acts as multimodal chromatographic ligand, thereby obtaining the chromatographic matrix used for the separation chromatographic assays - MP-SilPrMImCl. Both reactions described in stages 1) and 2) were refluxed during 24 h in an inert solvent - toluene -, and using triethylamine as the catalyst. The prepared matrix was broadly characterized by a set of complementary techniques, namely: - by ¹³C CPMAS, elemental analysis, and determination of PZC, to confirm the functionalization with the IL; - and by Scanning Electron Microscopy to evaluate the impact both of the functionalization and of the regeneration stage in the morphology of the synthesized matrix. Whenever justified and by way of comparison, in addition to the MP-SilPrMImCl matrix, the commercial macroporous matrix (MP), and MP-SilPrMImCl after several cycles of regeneration with aqueous solutions of sodium hydroxide (NaOH) and hydrochloric acid (HCI) were also characterized. Preliminary studies were conducted to evaluate the chromatographic behavior of the matrix MP-SilPrMImCl described in this disclosure, in what concerns its ability to bind a RNA sample, preferably a ImwRNA sample, obtained by extraction from a bacterial culture by the guanidinium thiocyanate/phenol/chloroform method. The injection and binding of the RNA sample, preferably a ImwRNA sample, in conditions of high concentration of ammonium sulfate [(NH₄)₂SO₄] was performed in order to mainly explore hydrophobic interactions. After the application of a stepwise gradient with a decreasing concentration of (NH₄)₂SO₄, it was observed that the RNA species did not elute at the time the ionic strength of the mobile phase was decreased; however, the coupling of a second elution step with an increase of the ionic strength employing sodium chloride (NaCI), has led the RNA species to elute. This behavior is typical of a multimodal matrix in which different types of interactions are established between the ligand and the target biomolecule. On the other hand, and using a distinct approach, the chromatographic matrix also showed ability to bind RNA, preferably ImwRNA, in conditions that mainly favor electrostatic interactions, i.e. with binding at low ionic strength conditions and elution through the increase in the NaCl concentration. It was also proved that the prepared matrix presents high-selectivity, given the ability to separate RNA from DNA, preferably to separate ImwRNA from gDNA, using a stepwise gradient with an increasing concentration of NaCl. According to this chromatographic strategy, RNA, preferably ImwRNA, is obtained in peak 1 which corresponds to the binding step, being therefore a negative chromatographic process that presents two major advantages: 1) the use of a lower salt concentration, which in addition to decrease the environmental footprint of the chromatographic process, also contributes to decrease the negative effect of the salt on maintaining RNA stability and integrity, 2) the decrease in the residence time of the sample in the chromatographic system, which also contributes to the maintenance of the stability of this biomolecule. In addition to the separation of ImwRNA and gDNA, the matrix MP-SilPrMImCl also exhibited the required selectivity to separate ImwRNA, rRNA and gDNA in conditions that favor electrostatic interactions, hereby confirming its high selectivity in the separation of nucleic acids from more complex bacterial lysates.

In general, the matrix MP-SilPrMImCl described in this disclosure demonstrated high versatility, the IL 1-methylimidazolium chloride being able to act as multimodal ligand in a nucleic acid separation chromatographic process characterized by high efficiency, specificity, robustness, and high dynamic binding capacity.

The present disclosure is referred to a chromatographic matrix which comprises:
a porous solid support,
a spacer covalently bound to the porous solid support,
a multimodal ligand covalently bound to the spacer, and
wherein the multimodal ligand is able to bind a nucleic acid.

In an embodiment, the multimodal ligand density, particularly the density of the immobilized multimodal ligand, in the porous solid support can vary between 0.01 and 2 mmol multimodal ligand/g of porous solid support, preferably between 0.5 and 1 mmol multimodal ligand/g of porous solid support, more preferred 0.726 mmol multimodal ligand per g of porous solid support.

In an embodiment, the molar ratio multimodal ligand:spacer can be 1:1.

In an embodiment, the multimodal ligand is an ionic liquid, for example, an ionic liquid selected from a family of imidazolium cations, pyridinium cations, piperidinium cations, quaternary ammonium cations or quaternary phosphonium cations, preferably combined with organic and inorganic anions.

In an embodiment, the ionic liquid can be selected from a family of imidazolium cations combined with an inorganic anion, preferably and in order to obtain improved results, the ionic liquid is 1-methylimidazolium chloride.

In an embodiment, the spacer can be selected from the following group: (3-chloropropyl)trimethoxysilane, (3-chloropropyl)triethoxysilane, (3-aminopropyl)trimethoxysilane, (3-mercaptopropyl)trimethoxysilane, (N-N-dimethylaminopropyl)trimethoxysilane or [3-(2-aminoethylamino)propyl]trimethoxysilane, preferably the spacer is (3-chloropropyl)trimethoxysilane.

In an embodiment and in order to obtain improved results, the spacer can be (3-chloropropyl)trimethoxysilane and the ionic liquid can be 1-methylimidazolium chloride.

In an embodiment, the porous solid support can be a polymer, preferably a polymer functionalized with epoxy groups, still more preferred a methacrylic polymer functionalized with epoxy groups, wherein said polymer can have a particle size between 40 and 90 µm, and a mean pore size of 1000 Å, these sizes being determined by techniques familiar to the skilled person in the art, as for example by scanning electron microscopy.

In an embodiment, the nucleic acid can be RNA and/or genomic DNA, preferably wherein the RNA can comprise low molecular weight RNA and/or ribosomal RNA.

The present disclosure also refers to a kit for nucleic acids separation or diagnostic, wherein said kit comprises the chromatographic matrix now disclosed.

The present disclosure still refers to a method for isolating nucleic acids from a sample wherein the method comprises the following steps:
contact the sample with the chromatographic matrix here described;
separately elute the nucleic acids from the sample.

In an embodiment, the sample can be a sample from a bacterial lysate that may comprise RNA and genomic DNA, preferably wherein the RNA may comprise low molecular weight RNA and/or ribosomal RNA.

In an embodiment, the step of separately eluting the nucleic acids can comprise separately eluting low molecular weight RNA and genomic DNA, preferably wherein the step of separately eluting the nucleic acids comprises separately eluting low molecular weight RNA and/or ribosomal RNA and/or genomic DNA.

In an embodiment, the step of separately eluting the sample nucleic acids can be performed with an increase in the ionic strength using a buffer with 10 mM Tris-HCI pH=8 and 1 M NaCl and following a stepwise gradient.

In an embodiment, the step of separately eluting the sample nucleic acids can comprise eluting low molecular weight RNA with 10 mM Tris-HCI pH=8 and 0.3 M NaCl - 0.4 M NaCl, preferably 0.38 M NaCl.

In an embodiment, the step of separately eluting the sample nucleic acids can comprise eluting genomic DNA with 10 mM Tris-HCI pH=8 and 0.5 M NaCl - 1 M NaCl, preferably 1 M NaCl.

In an embodiment, the step of separately eluting the sample nucleic acids can be performed by decreasing the concentration of 2 M of (NH₄)₂SO₄ in 10 mM Tris-HCI pH 8, following a stepwise gradient.

In an embodiment, the sample can be a sample from a bacterial lysate that can comprise low molecular weight RNA, genomic DNA and ribosomal RNA.

In an embodiment, the step of separately eluting the nucleic acids can comprise separately eluting low molecular weight RNA, genomic DNA and ribosomal RNA.

In an embodiment, the step of separately eluting the sample nucleic acids can be performed with an increase in the ionic strength using a buffer with Tris-HCl 10 mM pH=8 and 1 M NaCl and following a stepwise gradient.

In an embodiment, the step of separately eluting the sample nucleic acids can comprise eluting low molecular weight RNA with 10 mM Tris-HCI pH=8 and 0.3 M NaCl - 0.39 M NaCl, preferably 0.38 M NaCl.

In an embodiment, the step of separately eluting the sample nucleic acids can comprise eluting genomic DNA with 10 mM Tris-HCI pH=8 and 0.4 M NaCl - 0.45 M NaCl, preferably 0.41 M NaCl.

In an embodiment, the step of separately eluting the sample nucleic acids can comprise eluting ribosomal RNA with 10 mM Tris-HCI pH=8 and 0.46 M NaCl - 1 M NaCl, preferably 0.5 M NaCl.

The present disclosure also refers to a method for preparing the chromatographic matrix here described, wherein said method comprises the following steps:
covalently binding a spacer to a porous solid support;
covalently binding a multimodal ligand to the spacer of the previous step.

In an embodiment of the method for preparing the chromatographic matrix here described, the ionic liquid can be selected from a family of imidazolium cations, pyridinium cations, piperidinium cations, quaternary ammonium cations or quaternary phosphonium cations, preferably combined with organic or inorganic anions, preferably the ionic liquid is selected from a family of imidazolium cations combined with an inorganic anion, particularly the ionic liquid is 1-methylimidazolium chloride.

In an embodiment of the method for preparing the chromatographic matrix here described, the spacer can be selected from the following group: (3-chloropropyl)trimethoxysilane, (3-chloropropyl)triethoxysilane, (3-aminopropyl)trimethoxysilane, (3-mercaptopropyl)trimethoxysilane, (N-N-dimethylaminopropyl)trimethoxysilane or [3-(2-aminoethylamino)propyl]trimethoxysilane, particularly the spacer is (3-chloropropyl)trimethoxysilane.

In an embodiment of the method for preparing the chromatographic matrix here described, the spacer is (3-chloropropyl)trimethoxysilane and the ionic liquid is 1-methylimidazolium chloride.

In an embodiment of the method for preparing the chromatographic matrix here described, the porous solid support is a polymer, preferably a polymer functionalized with epoxy groups, still more preferred a methacrylic polymer functionalized with epoxy groups wherein the epoxylated methacrylic polymer has a particle size between 40 and 90 µm, and a mean pore size of 1000 Å.

This disclosure still refers to the use of the chromatographic matrix here described in a chromatographic process for isolating and/or purifying and/or separating nucleic acids, preferably for separating RNA from genomic DNA, still more preferred ImwRNA, rRNA and gDNA.

### Description of the Figures

For an easier understanding of the disclosure, figures are hereby attached, which represent preferred embodiments of the disclosure, which however are not intended to limit its subject-matter.
**Fig. 1****:** General procedure for the functionalization of the chromatographic matrix, preferably a macroporous matrix, particularly matrix of an epoxylated methacrylic polymer (Toyopearl® AF-Epoxy-650M, Tosoh Biosciences, GmbH, Greisheim, Germany), with the ionic liquid 1-methylimidazolium chloride.
**Fig. 2****:** ¹³C CPMAS NMR spectra of the chromatographic matrix, preferably a macroporous matrix, non-functionalized (MP), and the chromatographic matrix, preferably a macroporous matrix functionalized with the ionic liquid 1-methylimidazolium chloride (MP-SilPrMImCl), wherein any one of the macroporous matrices is a matrix of an epoxylated methacrylic polymer.

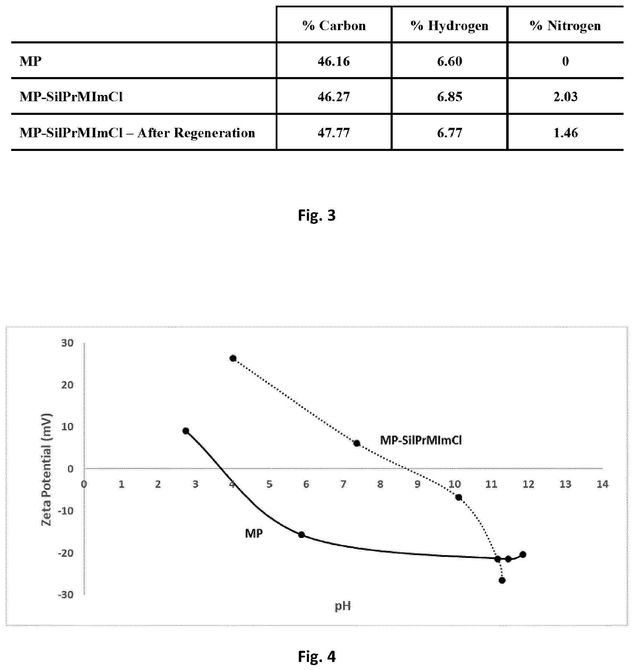

**Fig. 3****:** Register of PZC of suspensions of the non-functionalized chromatographic matrix (MP), preferably a non-functionalized macroporous matrix, and the functionalized chromatographic matrix, preferably a chromatographic matrix functionalized with the ionic liquid 1-methylimidazolium chloride (MP-SilPrMImCl) at different pH values, wherein any one of the macroporous matrices is a matrix of an epoxylated methacrylic polymer.
**Fig. 4****:** Elemental analysis of the non-functionalized macroporous matrix (MP), of the macroporous matrix functionalized with the ionic liquid 1-methylimidazolium chloride (MP-SilPrMImCI), and of the same matrix after 5 cycles of regeneration with aqueous solutions of sodium hydroxide and hydrochloric acid, respectively.

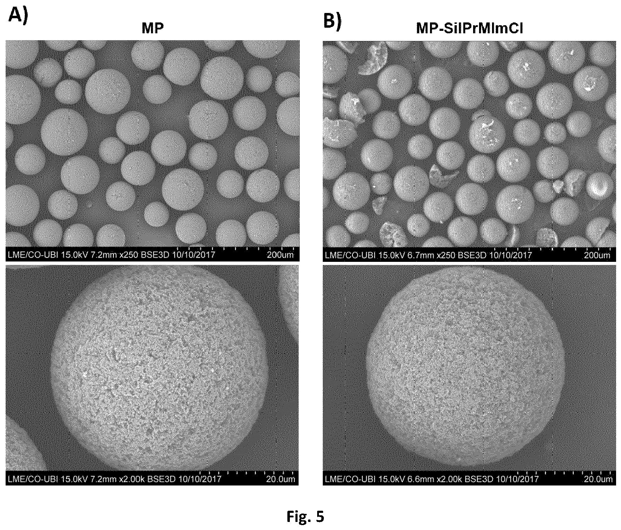

**Fig. 5****:** Scanning electron microscopy images: **A)** Macroporous matrix (MP), preferably a non-functionalized chromatographic matrix; **B)** Functionalized macroporous matrix, preferably a chromatographic matrix functionalized with the ionic liquid 1-methylimidazolium chloride (MP-SilPrMImCl). The images were collected with two distinct magnifications, 250 and 2000 x, wherein any one of the macroporous matrices is a matrix of an epoxylated mecthacrylic polymer.

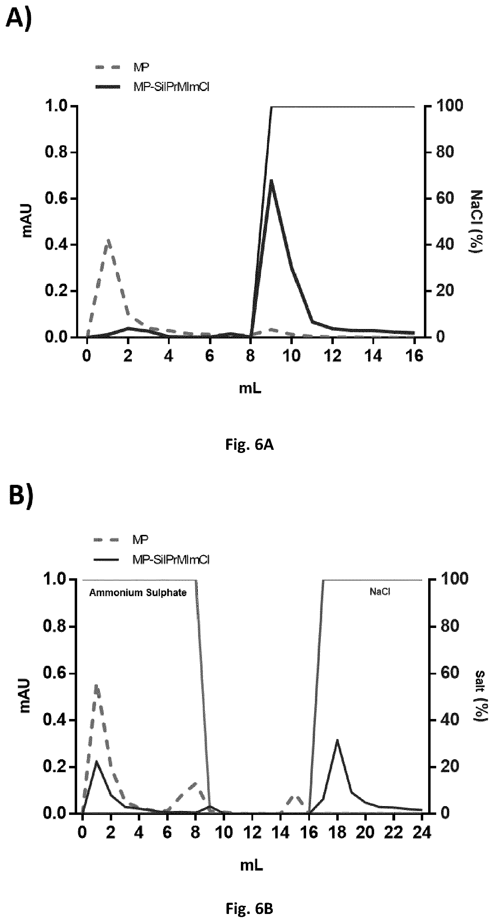

**Fig. 6****:** Chromatographic profiles of the retention of a sample of low molecular weight RNA in the macroporous chromatographic matrices, functionalized (MP-SilPrMImCl) and non-functionalized (MP) with the ionic liquid 1-methylimidazolium chloride, wherein any one of the macroporous matrices is a matrix of an epoxylated mecthacrylic polymer. **A)** Elution performed at 1 mL/min by an increase in the concentration of sodium chloride from 0 (10 mM Tris-HCI, pH 8) to 1 M, in 10 mM Tris-HCI at pH 8, by a stepwise gradient, as represented in the red line. **B)** Elution performed at 1 mL/min by a decreasing concentration of ammonium sulfate (2 M) to 0 M, i.e., 10 mM Tris-HCI, pH 8, by a stepwise gradient. A second elution step was performed by an increase in the concentration of sodium chloride to 1 M, in 10 mM Tris-HCI at pH 8, as represented in the red line. The sample of *Escherichia coli* low molecular weight RNA was obtained by extraction with the phenol-chloroform method, having been injected 40 micrograms of low molecular weight RNA in each assay.

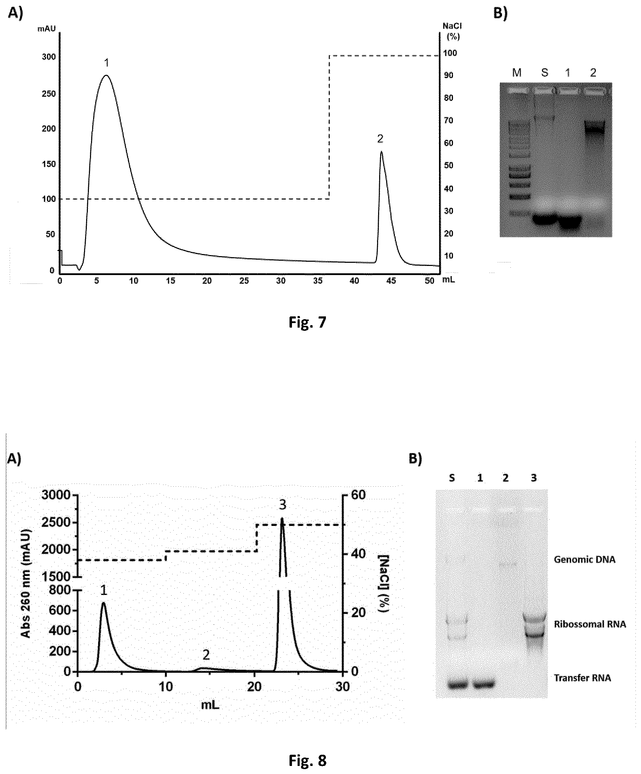

**Fig. 7****: A)** Chromatographic profile of the separation of genomic DNA and low molecular weight RNA with the functionalized chromatographic matrix, preferably macroporous matrix functionalized with the ionic liquid 1-methylimidazolium chloride (MP-SilPrMImCl). Elution performed at 1 mL/min by an increase in the concentration of sodium chloride, in 10 mM Tris-HCI at pH 8, by a stepwise gradient, as represented in the dashed line. The lysate sample from *Escherichia coli* containing genomic DNA and low molecular weight RNA was obtained employing the guanidinium thiocyanate method, having been injected 40 micrograms of sample in each assay. **B)** Schematic representation of agarose gel electrophoresis with analysis of the fractions collected from the chromatographic matrix MP-SilPrMImCl, preferably macroporous matrix MP-SilPrMImCl. M - Nucleic acids molecular weight marker; S - Lysate sample injected in the chromatographic matrix, preferably macroporous matrix; 1 - First chromatographic peak collected, representing the low molecular weight RNA; 2 - Second chromatographic peak collected, representing the genomic DNA.
**Fig. 8****: A)** Chromatographic profile of the separation of ImwRNA, rRNA and gDNA with the chromatographic matrix, preferably macroporous matrix, functionalized with ionic liquid 1-methylimidazolium chloride (MP-SilPrMImCl). Elution performed at 1 mL/min by an increase in the concentration of sodium chloride, in 10 mM Tris-HCI pH 8, as represented in the dashed line. The lysate sample from *Escherichia coli* comprising ImwRNA, rRNA and gDNA was prepared employing the guanidinium thiocyanate method, having been injected 40 micrograms of nucleic acids. **B)** Schematic representation of an agarose gel electrophoresis with the analysis of the fractions collected from the macroporous matrix MP-SilPrMImCl, wherein M represents the nucleic acids molecular weight marker; S represents the lysate sample injected in the macroporous/chromatographic matrix; 1 represents the first chromatographic peak to be collected, and corresponding to ImwRNA; 2 represents the second chromatographic peak to be collected, and corresponding to gDNA; 3 represents the third chromatographic peak to be collected, and corresponding to rRNA.

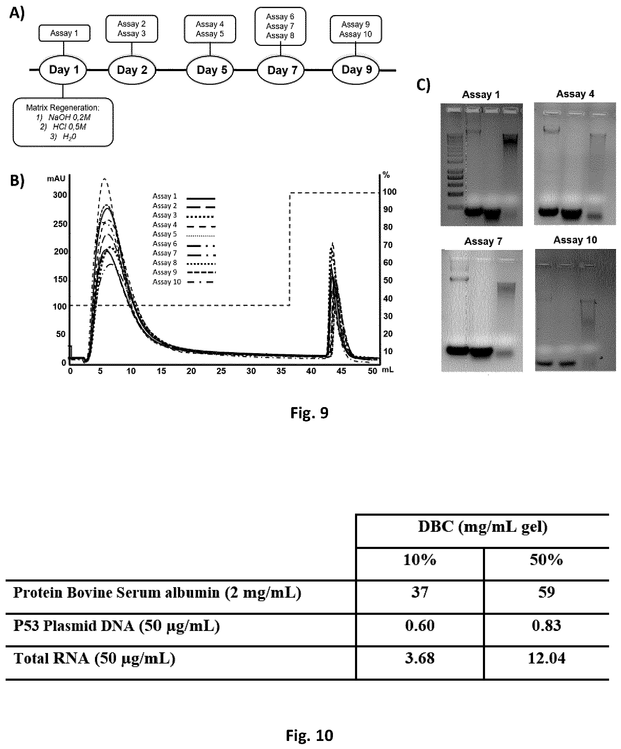

**Fig. 9****:** Evaluation of the reproducibility of the macroporous matrix functionalized with the ionic liquid 1-methylimidazolium chloride (MP-SilPrMImCl), preferably chromatographic matrix functionalized with the ionic liquid 1-methylimidazolium chloride (MP-SilPrMImCl) **A)** General procedure performed to evaluate the reproducibility encompassing the repetition of ten chromatographic assays, intercalated with regeneration steps with aqueous solutions of sodium hydroxide and hydrochloric acid. **B)** Chromatographic profiles of the ten assays performed, as represented by different colors. Elution performed at 1 mL/min by a stepwise gradient with an increase in the concentration of sodium chloride, in 10 mM Tris-HCI at pH 8, as represented by the dashed line. The lysate sample from *Escherichia coli* containing genomic DNA and low molecular weight RNA was obtained by extraction employing the guanidinium thiocyanate method, having been injected in each assay 40 micrograms of sample. **C)** Schematic representation of an agarose gel electrophoresis with the fractions collected from the macroporous matrix, preferably chromatographic, MP-SilPrMImCl in the assays one, four, seven and ten. S - Lysate sample injected in the macroporous matrix, preferably chromatographic; 1 - First chromatographic peak collected; 2 - Second chromatographic peak collected.
**Fig. 10****:** Dynamic binding capacity (mg/mL matrix) determination of the macroporous matrix, preferably chromatographic, functionalized with the ionic liquid 1-methylimidazolium chloride (MP-SilPrMImCl) for distinct biomolecules, namely, bovine serum albumin protein, plasmid DNA pcDNA3 flag p53 (Addgene, Cambridge, United States of America) and low molecular weight RNA. The capacity assays were performed at a flow-rate of 1 mL/min, with solutions of 2 mg/mL bovine serum albumin, 50 µg/mL p53 plasmid DNA, and 50 µg/mL low molecular weight RNA. Bovine serum albumin was commercially acquired, the p53 plasmid DNA was obtained through a commercial kit, and the low molecular weight RNA sample was obtained after extraction with the guanidinium thiocyanate-phenol-chloroform method.

### General description

The present disclosure encompasses the preparation of a novel multimodal chromatographic matrix with the IL 1-methylimidazolium chloride, obtained by immobilization of 1-methylimidazole in a macroporous stationary phase and using as spacer arm the (3-chloropropyl)trimethoxysilane, as well as its application in the separation of low molecular weight RNA from genomic DNA or in the separation of ImwRNA, rRNA and gDNA. The structure of the LI, namely with the presence of different functional groups, allows multiple interactions with the biomolecules to be established, and hydrophobic or electrostatic interactions can be favored, depending on the operation conditions. Its preparation involves the immobilization of (3-chloropropyl)trimethoxysilane in a macroporous matrix, particularly a matrix of an epoxylated methacrylic polymer, followed by reaction with 1-methylimidazole in order to obtain the matrix functionalized with the IL 1-methylimidazolium chloride. The separation of low molecular weight RNA from ribosomal RNA from genomic DNA was performed in conditions that mainly promote electrostatic interactions, involving the injection of the sample with 0.38 M NaCl (in 10 mM Tris-HCI, pH 8), followed by elution with 1 M NaCl (in 10 mM Tris-HCI, pH 8) by stepwise gradients, a step with 0.41 M NaCl and another step with 0.5 M NaCl, with the low molecular weight RNA, the genomic DNA and the ribosomal RNA being recovered with high-purity in peaks 1, 2 and 3, respectively. This matrix showed high robustness and reproducibility, as well as a high dynamic binding capacity for distinct biomolecules, assuming particular relevance in the preparation of RNA for therapeutic and diagnostic purposes or for general applications in molecular biology.

The present disclosure distances itself from all the works reported to date, thus presenting an innovative character not only due to the prepared chromatographic matrix, but also to the application for which it is intended, namely: 1) the IL 1-methylimidazolium chloride was used as multimodal ligand immobilized in a commercial macroporous matrix, particularly a matrix of an epoxylated methacrylic polymer; 2) the macroporous matrix, preferably the chromatographic matrix, particularly the polymeric matrix of epoxylated methacrylic, functionalized with 1-methylimidazolium chloride was used for the separation of nucleic acids from a bacterial extract, in a context of preparative chromatography; 3) (3-chloropropyl)trimethoxysilane was used as the spacer arm.

The present disclosure refers to the preparation of a novel chromatographic matrix using the IL 1-methylimidazolium chloride as multimodal ligand, as well as its application in the context of preparative chromatography, namely, in the separation of nucleic acids from a bacterial lysate. The preparation of matrix MP-SilPrMImCl encompassed two main stages: 1) Covalent bond of the spacer arm (3-chloropropyl)trimethoxysilane (Acros Organics, Thermo Fisher Scientific, Geel, Belgium) to the commercial macroporous matrix Toyopearl® AF-Epoxy-650M (Tosoh Biosciences GmbH, Greisheim, Germany), thus obtaining the matrix MP-SilPrCl; 2) Reaction of matrix MP-SilPrCl with 1-methylimidazole (Acros Organics, Thermo Fisher Scientific, Geel, Belgium) to form the multimodal ligand 1-methylimidazolium chloride in the surface of MP matrix. The process of synthesis of the matrix is described in **Fig. 1****,** as well as all the experimental details wherein the reactions were carried out. The characterization of the chromatographic matrix MP-SilPrMImCl was performed employing different techniques with the aim of confirming whether the IL was immobilized, or otherwise if the functionalization process induced alterations in different morphological parameters of the matrix. To this end, the following techniques were employed: ¹³C CPMAS, elemental analysis, PZC determination and scanning electron microscopy (SEM). The obtained results demonstrated that the IL was correctly immobilized in the prepared matrix. However, and despite no significant alterations were observed in the morphology of the spheres of the functionalized matrix in comparison with the commercial macroporous matrix, particularly a matrix of an epoxylated methacrylic polymer, it was found that the functionalized matrix presented some deformed spheres, which is due to the two processes of synthesis, filtration and drying. The regeneration of the chromatographic matrix is a procedure generally employed in preparative chromatography for matrix sanitization and removal of all kind of species that accumulate and that are not eluted in the successive assays. In this particular work, it is of special relevance once it is also important to reestablish the counter-ion chloride which is part of the IL 1-methylimidazolium chloride. Therefore, after several cycles of usage and regeneration, a characterization of the matrix was performed and, in general, no significant alterations were observed in comparison to the newly synthesized MP-SilPrMImCl matrix. The rationale behind the choice of the IL 1-methylimidazolium chloride as chromatographic ligand is related with its structure that comprises a hydrophobic aromatic ring, and a positive charge center with a chloride counter-ion. In this way, the nucleic acids - biomolecules negatively charged due to the phosphate groups, and moderately hydrophobic essentially due to the nitrogen bases -, interact by multiple non-covalent interactions, namely hydrophobic or electrostatic interactions, or both simultaneously. However, the different classes of nucleic acids show a different charge density, as well as a different degree of exposure of the nitrogen bases, which can result in distinct binding and elution chromatographic behaviors. This fact was explored with the purpose of proceeding to the isolation and separation of RNA and DNA, preferably separation of ImwRNA, rRNA and gDNA, from the elution thereof in conditions of distinct ionic strength. Indeed, the development of novel chromatographic strategies based on the application of novel ligands with improved selectivity, specificity, robustness and binding capacity for the purification of RNA is still highly desired in the (bio)pharmaceutical industry, namely, for obtaining RNA with high-purity, stability and quality suitable to their application in therapeutics, diagnostic, or in molecular biology-related procedures. Particularly, in order to avoid immune responses and other adverse effects, the quantity of gDNA that may be present in RNA samples, intended for therapeutic applications or in diagnostic methods should be reduced, in accordance with the requirements defined by the regulatory agencies for biopharmaceuticals such as pDNA. On the other hand, the presence of gDNA also negatively influences the accuracy of the results obtained in different molecular biology techniques carried out with RNA. These different factors emphasize the importance of developing novel methods for purifying RNA.

Overall, the procedure for obtaining the chromatographic matrix MP-SilPrMImCl and its application on the separation of RNA and gDNA, preferably ImwRNA, rRNA and gDNA, include the following steps:
1. Preparation of the macroporous chromatographic matrix Toyopearl® AF-Epoxy-650M (Tosoh Biosciences GmbH, Greisheim, Germany) with the IL 1-methylimidazolium chloride as chromatographic ligand, using 3-(chloropropyl)trimethoxysilane as spacer arm;
2. Characterization of the chromatographic matrix synthetized by different techniques: 1) ¹³C CPMAS, elemental analysis, and PZC determination to confirm the correct functionalization of the matrix with the ligand - **Figs. 2****,** **3** and **4****;** 2) Scanning electron microscopy to evaluate whether the functionalization process induces any alterations in the matrix morphology, represented in **Fig. 5****;**
3. Obtention of the nucleic acids sample from *Escherichia coli* (*E. coli*) DH5α culture, envisaging its subsequent application in separation assays in the matrix MP-SilPrMImCl. Bacterial growth was performed in specific experimental conditions, whereas the bacterial lysate containing ImwRNA and gDNA, or ImwRNA, rRNA and gDNA, resulted from the application of the lysis method based on guanidinium thiocyanate, while ImwRNA samples were obtained by the guanidinium thiocyanate-phenol-chloroform method, according to previously optimized and reported protocols;
4. Evaluation of the effect of the functionalization of the matrix MP with the IL 1-methylimidazolium chloride in the retention capacity of RNA, preferably ImwRNA, and characterization of the mechanism of interaction. These assays were performed in disposable columns, in which the matrices MP and MP-SilPrMImCl were individually packed and binding and elution assays were performed in conditions that mainly promote electrostatic interactions. The recovery of RNA, preferably ImwRNA, was promoted by the increase of the ionic strength in the mobile phase, associated to the increase of the NaCl concentration. In these assays a RNA sample was used, preferably a ImwRNA sample, obtained by the guanidinium thiocyanate-phenol-chloroform method. As demonstrated in **Fig. 6A****,** it was verified that matrix MP does not have the ability to retain RNA, preferably ImwRNA, unlike MP-SilPrMImCl matrix, hereby confirming that the chromatographic ligand 1-methylimidazolium chloride is the responsible for the interaction with the biomolecule. On the other hand, experiments in conditions that mainly favor hydrophobic interactions were performed, particularly with the use of a high ionic strength-mobile phase as binding condition and with a decrease of the ionic strength by decreasing the (NH₄)₂SO₄ (in Tris-HCI 10mM, pH 8) concentration by a stepwise gradient, for elution. However, after application of this first elution condition with 10 mM Tris-HCI, pH 8, it was found that the majority of RNA species, preferably ImwRNA, still remained adsorbed to the matrix, being only eluted with a second elution step with 1 M NaCl (in 10 mM Tris-HCI, pH 8). These observations indicate that the interaction established between ligand-biomolecule is not due to solely one type of interaction but instead results from the combination of several interactions. Therefore, depending on the experimental conditions used, is possible to explore the multimodal character of the matrix MP-SilPrMImCl wherein different interactions seem to occur simultaneously;
5. Separation of RNA, preferably ImwRNA, from gDNA by multimodal chromatography, using the chromatographic matrix MP-SilPrMImCl. The results obtained in point 4 of this section, wherein it was reported that the matrix MP-SilPrMImCl shows a multimodal behavior capable of establishing different types of interactions with biomolecules, agree well with the structure of the chromatographic ligand. Indeed, 1-methylimidazolium chloride presents an aromatic ring, exhibiting: 1) high hydrophobicity, allowing the establishment of hydrophobic interactions; 2) a positive charge center, allowing the establishment of electrostatic interactions with negatively charged biomolecules, thus acting as anion exchanger with the chloride initially present as counter-ion. However, due to the negative impact in the environment of using high salt concentrations, the separation between RNA, preferably ImwRNA, and gDNA in the matrix MP-SilPrMImCl was performed in conditions that mainly favor electrostatic interactions. After successive optimizations, a strategy was developed that allows the separation of RNA, preferably ImwRNA, from gDNA, wherein RNA, preferably ImwRNA, is recovered in the binding step in a high purity state, as shown in **Fig. 7****.** This process is typically termed as negative chromatography, once the biomolecule of interest is recovered in the binding condition, being thus valuable for the maintenance of the stability of RNA, once in addition to a lower residence period in the system, it is recovered with a lower salt concentration;
6. Separation of ImwRNA, rRNA and gDNA by multimodal chromatography using the chromatographic matrix MP-SilPrMImCl. As highlighted in point 4, although the matrix MP-SilPrMImCl is able to establish different types of interactions with biomolecules, the use of high salt concentrations usually applied to promote mainly hydrophobic interactions has a negative impact in environment. Therefore, the separation of ImwRNA, rRNA and gDNA was accomplished in conditions that mainly promote electrostatic interactions, namely, an increasing NaCl gradient. After successive optimizations, a strategy was developed that allows the separation of ImwRNA, rRNA and gDNA, wherein ImwRNA, gDNA and rRNA are recovered with high-purity in peaks 1, 2 and 3, respectively, according to **Fig. 8****.**
7. Evaluation of the reproducibility of the matrix MP-SilPrMImCl in the separation of RNA, preferably ImwRNA, and gDNA. The chromatographic behavior of the matrix described in this disclosure was evaluated after diverse cycles of chromatographic assays, intercalated with regeneration processes;
8. Determination of the dynamic binding capacity of the chromatographic matrix MP-SilPrMImCl for different biomolecules. The dynamic binding capacity was individually determined for 3 distinct biomolecules, the bovine serum albumin protein (BSA), plasmid DNA (pDNA), and RNA, preferably ImwRNA, using for the purpose solutions of said biomolecules with concentrations of 2 mg/mL, 50 µg/mL, e 50 µg/mL, respectively. To that end, the equilibrium of the chromatographic support was carried out with 10 mM Tris-HCI (pH 8), the respective solutions of each biomolecule (dissolved in 10 mM Tris-HCI, pH 8) were injected, and finally, the elution of retained biomolecules was performed by the increase in the concentration of NaCl (in 10 mM Tris-HCI, pH 8) by a stepwise gradient. The determination of the dynamic binding capacity values for BSA, pDNA and ImwRNA was performed at 10 and 50 % of the maximum capacity value.

### Detailed description

The present disclosure refers to the development of a novel chromatographic matrix, obtained through the functionalization of a commercial macroporous stationary phase with the IL 1-methylimidazolium chloride (MP-SilPrMImCI), aiming the separation between RNA, preferably ImwRNA, and gDNA from a bacterial extract. The preparation of this chromatographic matrix and the subsequent application in separation processes of nucleic acids, comprise the following steps:

1. Functionalization of the macroporous chromatographic matrix Toyopearl® AF-Epoxy-650M (Tosoh Biosciences GmbH, Greisheim, Germany) with the IL 1-methylimidazolium chloride (Acros Organics, Thermo Fisher Scientific, Geel, Belgium), as described in **Fig. 1****.** Initially, the spacer arm was covalently bound - (3-chloropropyl)trimethoxysilane - (Acros Organics, Thermo Fisher Scientific, Geel, Belgium) to the commercial chromatographic matrix. Thus, a suspension of 5 g of the macroporous matrix (MP) was prepared in 60 mL of toluene, to which were added 5 mL of (3-chloropropyl)trimethoxysilane, and 0.5 mL of triethylamine, used as reaction catalyst. This suspension was refluxed during 24 h under mechanical stirring (550 rotations per minute - rpm). After this period, the suspension was cooled down to room temperature, the solid was separated by filtration and was subsequently washed with diverse solvents, namely: toluene, ethanol-water (1:1), bidistilled water and methanol. The solid was dried at 60 °C during 24 h, yielding the matrix MP with the spacer arm (MP-SilPrCl), that was vacuum-dried during 24 h at 60 °C. Then, the matrix MP-SilPrCl was reacted with 1-methylimidazole, aiming to obtain the matrix MP functionalized with the IL 1-methylimidazolium chloride. To a suspension of 5g of matrix MP with the spacer (MP-SilPrCl) in 60 mL of toluene, were added 5 mL of 1-methylimidazole. This suspension was refluxed during 24h under magnetic stirring, particularly 550 rpm. After this period, the solid was filtrated and washed with methanol, distilled water and methanol, and was then dried at 60 °C, thus yielding the chromatographic matrix functionalized with the IL (MP-SilPrMImCl).

In an embodiment, the chromatographic matrix MP-SilPrMImCl was characterized by ¹³C CPMAS, elemental analysis, PZC determination and scanning electron microscopy. The characterization of the matrix by ¹³C NMR was performed at room temperature with a VTN probe operating at 100.6 MHz, having been used the following CPMAS NMR ¹³C settings, in order to maximize the signal-to-noise ratio: *v*₁¹³ C = 55 kHz; recycle delay, 4 s; contact time, 1-2 ms; NS = 1k; *v*R = 12 kHz. The sequence SPINAL-64 decoupling was used during experimental data acquisition. According **Fig. 2****,** through the comparison of the NMR spectra obtained from ¹³C in the solid state for the functionalized (MP-SilPrMImCl) and non-functionalized (MP) matrices, it was detected the presence of peaks with chemical shifts between 120 and 130 ppm and between 35 and 40 ppm in the spectrum corresponding to matrix MP-SilPrMImCl, and which are not present in the spectrum of matrix MP. The signal with chemical shift at 37 ppm corresponds to the methyl group as alkyl side chain of the imidazolium cation whereas the signals at 120 - 130 ppm are characteristic of aromatic carbon atoms, which are present in the aromatic ring of imidazolium. In general, these results confirm the correct functionalization of the chromatographic matrix, preferably macroporous matrix, with the IL 1-methylimidazolium chloride, once these chemical shifts are not observed in the spectrum corresponding to the matrix MP-SilPrCl. The elemental analysis was performed to quantitatively determine the content in carbon, hydrogen and nitrogen in matrices MP and MP-SilPrMImCl. To this end, the equipment TruSpec 630-200-200 and approximately 2 mg of each sample were used. Additionally, the combustion furnace temperature was set to 1075 °C, the afterburner temperature to 850 °C, and the infrared absorption method was used for carbon and hydrogen quantification, while the thermal conductivity was selected for nitrogen. According the results described in **Fig. 3****,** it is observed that the MP matrix presented a content in carbon, hydrogen and nitrogen (Mass percentage, %) of 46.16, 6.60 and 0 %, respectively. On the other hand, the functionalized matrix MP-SilPrMImCl presents a mass % in carbon, hydrogen and nitrogen of 46.27, 6.85 and 2.03 %, respectively, wherein nitrogen comes exclusively from the IL 1-methylimidazolium chloride, indicating that the matrix was correctly functionalized. Moreover, the elemental analysis of the matrix MP-SilPrMImCl was performed after 5 chromatographic assays, intercalated with 5 cycles of regeneration with aqueous solutions of NaOH and HCI and washing with bidistilled water, and it was observed that the content in carbon and hydrogen was similar to matrices MP and non-regenerated MP-SilPrMImCl. However, a slightly lower value for the nitrogen content was obtained, in comparison with the non-regenerated MP-SilPrMImCl matrix, what can indicate that after diverse cycles of usage, the covalent bond of some ligand molecules can be broken, resulting into a slight co-elution of the ligand. For the determination of PZC of MP matrix and MP-SilPrMImCl matrix, the values of PZC of the suspensions of these materials in distilled water at different pH values (adjusted with 0.1 M HCI and NaOH solutions) were recorded. It was observed that the matrices MP and MP-SilPrMImCl presented a null PZC at pH values of approximately 3.7 and 8.7, respectively, what once again confirms the correct functionalization of the matrix MP with IL 1-methylimidazolium chloride.

In an embodiment, it was verified by scanning electron microscopy that the functionalization of matrix MP with the IL did not induce significative changes in its morphology, according to **Fig. 5****.**

In an embodiment, the sample of nucleic acids was obtained from a fermentative process of *Escherichia coli* DH5α. The bacterial cellular growth was performed in *erlenmeyer* flasks in a suitable culture medium (12 g/L tryptone, 24 g/L yeast extract, 4 mL/L glycerol, 0.017M KH₂PO₄, 0.072 M K₂HPO₄) under specific conditions of orbital rotation (250 rpm), and temperature (37 °C) and using a fermentation period of 2 hours aiming to maximize the production of ImwRNA, rRNA, gDNA and 7 hours for the production of ImwRNA and gDNA. Then, the obtention of a sample of bacterial lysate containing RNA, preferably ImwRNA, and gDNA or RNA, preferably ImwRNA and rRNA, was performed after the application of a process of cellular lysis and nucleic acids recovery. Initially, cell lysis was performed employing the solution that contains the guanidinium thiocyanate salt, followed by a concentration step with isopropanol, a washing step with ethanol, and a solubilization step with 0.05 % diethyl pyrocarbonate (DEPC)-treated bidistilled water, this constituting the nucleic acids sample which was applied for the separation studies of the functionalized chromatographic matrix.

In an embodiment, the evaluation of the effect of the functionalization of the matrix MP with the IL 1-methylimidazolium chloride to retain a RNA sample, preferably ImwRNA, and characterization of the mechanism of interaction were performed. It was studied the binding profiles to matrices MP and MP-SilPrMImCl of a RNA sample, preferably ImwRNA, isolated from a bacterial extract using the extraction method based on guanidinium thiocyanate-phenol-chloroform. For performing these assays, 2 mL of hydrated MP and MP-SilPrMImCl matrices were individually packed in polypropylene disposable columns (Econo-Pac®, Bio-rad, California, USA). Subsequently, assays were performed in experimental conditions that mainly favor electrostatic interactions, more specifically, the above-mentioned chromatographic matrices were equilibrated with 10 mM Tris-HCI pH 8 buffer, the RNA sample, preferably ImwRNA, was injected in the same equilibrium buffer and, finally, the retained RNA species were eluted by the increase in the ionic strength, i.e., in NaCl concentration to 1 M (in 10 mM Tris-HCI, pH 8) using a stepwise gradient. Samples from the fractions obtained during binding of the sample, and during elution were collected, the absorbance at 260 nm (A₂₆₀) was measured, and the respective chromatograms were drawn, as represented in **Fig. 6A****.** Indeed, it is observed that in the non-functionalized matrix - MP -, only a residual binding of RNA species occurred, as indicated by the approximately null absorbance at A₂₆₀, when the NaCl concentration in the elution buffer was increased to 1 M. On the other hand, the opposite profile was obtained in the matrix MP-SilPrMImCl, i.e., an approximately null A₂₆₀ in the binding step - 10 mM Tris-HCI pH 8 - and was observed a significant increase in A₂₆₀ with the elution buffer - 1 M NaCl in 10 mM Tris-HCI pH 8 -, indicating that the totality of RNA species interacted with the matrix. Thus, it is proved that the IL 1-methylimidazolium chloride effectively acts as chromatographic ligand, and is the species responsible for the interaction with nucleic acids, since the non-modified macroporous matrix presents a negligible ability to bind the low molecular weight RNA.

Aiming characterizing the interactions involved in the adsorption of RNA, preferably ImwRNA, to the novel matrix described in this disclosure, chromatographic assays were carried out in experimental conditions different than those aforementioned, that mainly favor hydrophobic interactions. To this end, the matrices MP and MP-SilPrMImCl were initially equilibrated with 2 M (NH₄)₂SO₄ (in Tris-HCI 10mM, pH 8), the RNA sample, preferably ImwRNA, was injected in the same equilibrium buffer, and finally, the retained RNA species were eluted by a decrease in the ionic strength to 10 mM Tris-HCI, using a stepwise gradient. As previously described, fractions from the binding and elution steps were collected and A₂₆₀ was determined. According to **Fig. 6B****,** after the first elution step with 10 mM Tris-HCI pH 8, it was observed that the majority of RNA species, preferably ImwRNA, remained retained in the matrix. Thus, a second elution step with 1 M NaCl (in 10 mM Tris-HCI, pH 8) was performed, and it was verified that the RNA species, preferably ImwRNA, were effectively eluted, according to the A₂₆₀ values recorded in **Fig. 6B****.** The chromatographic behavior exhibited by matrix MP-SilPrMImCl in these experimental conditions is typically characteristic of multimodal matrices, i.e., stationary phases in which more than one type of interactions between the biomolecule and the chromatographic ligand are established. Therefore, the chromatographic behavior of matrix MP-SilPrMImCl described in this disclosure was characterized, which acts mostly as an anion exchanger in conditions that mainly favor electrostatic interactions, but that in general presents a multimodal behavior, namely due to the structural characteristics of the IL 1-methylimidazolium chloride as ligand.

In an embodiment, the separation of RNA, preferably ImwRNA, from gDNA by multimodal chromatography, using the chromatographic matrix MP-SilPrMImCl was analyzed. According to the structure of the chromatographic ligand immobilized in the functionalized matrix - IL 1-methylimidazolium chloride -, it was verified that it exhibits an aromatic ring, which: 1) presents high hydrophobicity, making the establishment of hydrophobic interactions possible; 2) also presents a positive charge center, allowing the establishment of electrostatic interactions with negatively charged biomolecules. In particular, nucleic acids present in its constitution negatively charged phosphate groups, and nitrogen bases with hydrophobic character, which enables its interaction with chromatographic matrices by electrostatic and hydrophobic interactions, respectively. Multimodal chromatography explores different types of interactions between the ligand immobilized in the stationary phase and the target biomolecule, wherein the cooperativity of these interactions generally leads to an increase in the selectivity and specificity of the chromatographic process. However, through the proper choice of binding and elution experimental conditions, the matrix may also be able to operate in unique mode, thus allowing to explore mainly one type of interactions, making the matrices more versatile for any separation. The mechanism of interaction between a RNA sample, preferably ImwRNA, and the matrix MP-SilPrMImCl was characterized in point 4) of this section, wherein can be established predominantly electrostatic interactions, hydrophobic interactions or multiple interactions (electrostatic, hydrophobic, hydrogen bonds, cation-π interactions, and van der Waals forces), depending on the experimental conditions employed. However, the high eutrophication potential of (NH₄)₂SO₄, along with the fact that it is obtained from non-renewable sources, causes the use of high concentrations of this reagent to have a negative impact on the environment, and such reason led to explore mainly the anion-exchanger character of the matrix prepared for the separation of nucleic acids. Thus, a 10 mm diameter and 26 mm height chromatographic column (Ge Healthcare, Uppsala, Sweden) was packed with 2 mL of hydrated matrix MP-SilPrMImCl, and chromatographic assays were performed in the ÄKTA Avant system under the control of *software* Unicorn 6 (Ge Healthcare, Uppsala, Sweden), which allows to automatically collect the different fractions, and on-line controlling diverse parameters, such as conductivity, absorbance, and pH. Moreover, a thermostatized column was used and the room temperature was set as the operation temperature - 25 °C. Initially, a bacterial lysate sample composed of ImwRNA and gDNA was used and after performing different assays, the optimized strategy consisted in the equilibrium of MP-SilPrMImCl matrix with 0.380 M NaCl (in 10 mM Tris-HCI, pH 8), followed by the increase in the concentration of NaCl to 1 M by a stepwise gradient, thereby obtaining 2 chromatographic peaks, which are represented in **Fig. 7A** by 1 and 2, respectively. The samples represented by 1 and 2 were separately collected, concentrated and dessalted using vivaspin concentrators (5000 MWCO, Sartorius, Gottingen, Germany), and were finally analyzed in a 1 % (m/v) agarose gel, containing GreenSafe Premium (Nzytech, Lisbon, Portugal) at a concentration of 0.014 µL/mL of agarose gel. The obtained results are described in **Fig. 7B****,** where it is possible to observe two bands in the initial sample - Fraction S - which correspond to distinct species of nucleic acids, namely, gDNA with a higher molecular weight, and RNA, preferably ImwRNA, with a lower molecular weight. On the other hand, it was verified that RNA, preferably ImwRNA, elutes in peak 1, while in peak 2 only elutes gDNA, which confirms that the chromatographic strategy implemented and developed with MP-SilPrMImCl matrix allows an effective separation of RNA, preferably ImwRNA, from gDNA, from the lysis of a bacterial extract. This strategy is termed as negative chromatography, since the biomolecule of interest, hereby the RNA, preferably ImwRNA, is recovered in peak 1 - binding step -, whereas the remaining contaminants interact with the matrix, being subsequently recovered. Thus, this chromatographic process presents two unequivocal advantages, in addition to the biomolecule of interest being recovered with a lower NaCl concentration, the residence time in the chromatographic system is much lower, which contributes to maintain its stability, a factor which is crucial since RNA is a highly unstable biomolecule and susceptible to degradation by ribonucleases. Subsequently, a more complex bacterial lysate sample, composed of ImwRNA, rRNA and gDNA, was applied in matrix MP-SilPrMImCl. After successive experiments, the optimized purification strategy consisted in the equilibrium of MP-SilPrMImCl matrix with 0.380 M NaCl (in 10 mM Tris-HCI pH 8), followed by two elution steps through a stepwise gradient aiming the increase in the concentration of NaCl to 0.41 M and 0.5 M (in 10 mM Tris-HCI, pH 8). As a result, in addition to the peak corresponding to the species that did not bind to the matrix, 2 additional chromatographic peaks were obtained, which are represented in **Fig. 8A** by 1, 2 and 3, respectively. The samples represented by 1, 2 and 3 were treated as previously described and analyzed by agarose gel electrophoresis. The obtained results are shown in **Fig. 8B****,** and it is possible to observe 4 main bands in the initial sample - Fraction S - which correspond to distinct species of nucleic acids, namely the higher molecular weight band which corresponds to gDNA, the 2 bands with intermediate molecular weight corresponding to rRNA and the forth band which corresponds to ImwRNA and which presents the lower molecular weight. On the other hand, it was verified that highly purified nucleic acids were eluted in distinct peaks, namely, ImwRNA in peak 1, gDNA in peak 2 and rRNA in peak 3, hereby confirming that the chromatographic strategy implemented and developed with the MP-SilPrMImCl matrix allows an effective separation of ImwRNA, rRNA and gDNA, present in a bacterial extract.

In an embodiment, the evaluation of the reproducibility of MP-SilPrMImCl in the separation of RNA, preferably ImwRNA, from gDNA was performed as follows. 10 Chromatographic assays were conducted in 5 non-consecutive and different days, namely days 1, 2, 5, 7, and 9, and were intercalated with a regeneration process that comprises the contact of the chromatographic matrix with an aqueous solution of 0.2 M NaOH, bidistilled water, 0.5 M HCI, and bidistilled water, according to the scheme in **Fig. 9A****.** The chromatograms corresponding to the 10 performed assays are represented in **Fig. 9B****,** and the analysis by agarose gel electrophoresis of the peaks obtained in assays 1, 4, 7 and 10 corresponds to **Fig. 9C****.** In general, it was verified that the chromatographic matrix MP-SilPrMImCl is robust and reproducible, with the RNA, preferably ImwRNA, being recovered in peak 1 in a high-purity state in all the assays performed. However, it was verified that with the increase in the number of chromatographic assays performed, the genomic DNA is obtained in peak 2 slightly contaminated with RNA, preferably ImwRNA. Yet, although a slight decrease in the recovery levels of low molecular weight RNA in peak 1 was observed, no alterations were detected in its purity.

In an embodiment, the dynamic binding capacity of the chromatographic matrix MP-SilPrMImCl was determined as follows. The dynamic binding capacity for a particular biomolecule plays a paramount role in the characterization and evaluation of a chromatographic process, and is defined as the maximum amount of biomolecule that binds to the support under specific operation conditions. The binding capacity was individually determined for 3 distinct biomolecules, the bovine serum albumin (BSA), plasmid DNA (pDNA) and ImwRNA, using for the purpose solutions of said biomolecules with concentrations of 2 mg/mL, 50 µg/mL, and 50 µg/mL, respectively. The chromatographic process conducted to determine the dynamic binding capacity consisted: 1) in the equilibrium of the chromatographic matrix with 10 mM Tris-HCI (pH 8), in order to allow the binding of each different species; 2) in the injection of the respective solutions of each biomolecule (dissolved in 10 mM Tris-HCI, pH 8), and, finally, 3) in the elution of the retained biomolecules by the increase in the NaCl concentration (in 10 mM Tris-HCI, pH 8) by a stepwise gradient. The determination of the dynamic binding capacity values for BSA, pDNA and total RNA was performed at 10% and 50% of the maximum capacity value, and these values are reported in **Fig. 10****.**

### Application example 1:

In an embodiment, the determination of the retention of a RNA sample, preferably ImwRNA, in the matrix MP-SilPrMImCl in distinct chromatographic conditions, and characterization of the mechanism of interaction was performed as described below.

The chromatographic matrix described in this disclosure was obtained from the commercial macroporous matrix Toyopearl® AF-Epoxy-650M, in which a spacer arm was immobilized in a first instance, and 1-methylimidazole was then bound to the spacer arm, thereby leading to the formation of the multimodal chromatographic ligand. Initially, envisaging the covalent bonding of the spacer arm to the commercial matrix, a suspension of 5g of MP matrix was prepared in 60 mL toluene to which 5 mL of (3-chloropropyl)trimethoxysilane and 0.5 mL of triethylamine (with the function of catalyzing the reaction) were added. After refluxing 24h and under magnetic stirring, the suspension was cooled down, the solid isolated by filtration and washed with toluene, ethanol/water (1:1), distilled water and, finally, with methanol. The matrix with the spacer is thus obtained and is designated as MP-SilPrCl. Subsequently, the matrix MP-SilPrCl was reacted with 1-methylimidazole aiming to obtain the MP functionalized with the IL 1-methylimidazolium chloride. To a suspension of 5 g of matrix MP with the spacer (MP-SilPrCl) in 60 mL of toluene, 5 mL of 1-methylimidazole were added. This suspension was refluxed during 24 h under magnetic stirring (550 rpm). Subsequently, the solid was filtrated and washed with methanol, distilled water and methanol and dried at 60 ºC, thereby yielding the chromatographic matrix functionalized with the IL (MP-SilPrMImCl).

The chromatographic matrix functionalized with the IL (MP-SilPrMImCl) was characterized using distinct techniques: ¹³C CPMAS, elemental analysis, PZC determination, and SEM. In what concerns to the analysis by NMR-CPMAS, specific settings were applied in order to maximize the signal-noise ratio: *v*₁¹³ C = 55 kHz; *recycle delay,* 4 s; contact period, 1-2 ms; NS = 1k; vR = 12 kHz. The ¹³C CPMAS spectra of matrices MP and MP-SilPrMImCl are represented in **Fig. 2****,** and the presence of peaks with chemical shifts within the intervals 120-130 and 35-40 ppm was observed in the MP-SilPrMImCl spectrum, which are not present in matrix MP. If on one hand, the signal with chemical shift at 37 ppm is due to the presence of the methyl group as alkyl chain of the imidazolium, the signals at 120-130 ppm are characteristic of the aromatic carbon atoms, which are solely present in the imidazolium aromatic ring. In addition, the elemental analysis of MP and MP-SilPrMImCL matrices was performed to quantitatively determine the content in carbon, hydrogen and nitrogen present therein, according to **Fig. 3****.** In general, nitrogen was detected in matrix MP-SilPrMImCl, which is not present in the commercial macroporous matrix, confirming the results obtained by ¹³C CPMAS on the correct functionalization of the prepared matrix. The superficial charge of matrices MP and MP-SilPrMImCl was also evaluated by determining the PZC at different pH values. As represented in **Fig. 4****,** it was verified that the pH at which the matrices present a PZC, respectively, 3.7 and 8.7, for matrices MP and MP-SilPrMImCl, this change being due to the presence of IL 1-methylimidazolium chloride. Finally, after the analysis of **Fig. 5****,** through scanning electron microscopy it was verified that the functionalization of matrix MP with the IL did not induce significant alterations on its morphology.

The RNA sample, preferably ImwRNA, required for determining the retention profiles in the synthesized matrix was obtained through a procedure that comprises two steps: 1) biomass accumulation by *Escherichia coli* DH5α fermentation in a suitable culture medium (12g/L tryptone, 24g/L yeast extract, 4mL/L glycerol, 0.017M KH₂PO₄, 0.072M K₂HPO₄) under specific conditions of orbital rotation (250 rpm), and temperature (37 °C) during 7 hours; and 2) isolation of RNA, preferably ImwRNA, from the bacterial extract using the extraction method based on guanidinium thiocyanate-phenol-chloroform.

In an embodiment, to evaluate the capacity of the prepared matrix to retain a RNA sample, preferably ImwRNA, 2 mL of the matrix were packed in a polypropylene disposable column (Econo-Pac®, Bio-rad, California, USA). Subsequently, assays were performed in distinct experimental conditions: 1) using a gradient based on the increase in NaCl concentration, favoring mainly electrostatic interactions; 2) using a gradient based on the decrease in (NH₄)₂SO₄ concentration, where hydrophobic interactions are preferably established. To perform the assay 1, the chromatographic matrix was initially equilibrated with 10 mM Tris-HCI pH 8 buffer, the RNA sample, preferably ImwRNA, was injected in the same equilibrium buffer and, finally, the retained RNA species were eluted by the increase in the ionic strength, namely, the NaCl concentration to 1 M (in 10 mM Tris-HCI, pH 8) using a stepwise gradient. Samples from binding and elution steps were collected, the A₂₆₀ was determined, and the corresponding chromatogram was drawn. According to **Fig. 6A****,** there is an approximately null A₂₆₀ in the binding step - 10 mM Tris-HCI pH 8 -, and a significant increase in A₂₆₀ was achieved with the elution buffer - 1M NaCl in 10 mM Tris-HCI, pH 8 -, indicating that almost all RNA species interacted with the matrix.

In an embodiment, to explore mainly hydrophobic interactions between the ligand and the RNA sample, preferably ImwRNA, the chromatographic matrix was initially equilibrated with 2 M (NH₄)₂SO₄ in 10 mM Tris-HCI, pH 8, the RNA sample, preferably ImwRNA, was injected in the same equilibrium buffer and, finally, the retained RNA species were eluted by a decrease in (NH₄)₂SO₄ concentration to 10 mM Tris-HCI, pH 8, in stepwise gradient mode. Similar to what described above, samples were collected and A₂₆₀ was determined to draw the chromatogram, according to **Fig. 6B****.** Effectively, after elution with 10 mM Tris-HCI, pH 8, it was verified that the majority of RNA species were still retained in the matrix, and therefore a second elution with 1 M NaCl (in 10 mM Tris-HCI, pH 8) was performed. With this elution step, it was verified that RNA species, preferably ImwRNA, eluted. From this assay, it is possible to infer that multiple interactions between RNA, preferably ImwRNA, and the chromatographic ligand 1-methylimidazolium chloride were established, including electrostatic and hydrophobic interactions. This behavior is typical of multimodal chromatographic matrices where multiple interactions are established.

In general, the matrix MP-SilPrMImCl described in this disclosure presents the ability to retain RNA. It acts as an anion-exchanger in conditions that mainly favor electrostatic interactions, but presents a multimodal behavior more pronounced in conditions that mainly favor hydrophobic interactions. Therefore, the IL 1-methylimidazolium chloride is here presented as a multimodal ligand.

### Application example 2:

In an embodiment, the separation of RNA, preferably ImwRNA, from gDNA by multimodal chromatography, using the matrix MP-SilPrMImCl was performed as described below.

In an embodiment, the chromatographic matrix described in this disclosure was obtained from the commercial macroporous matrix Toyopearl® AF-Epoxy-650M, in which a spacer arm was immobilized in a first instance, and then 1-methylimidazole was bound to the spacer arm, thereby leading to the formation of the chromatographic ligand. Initially, envisaging the covalent bonding of the spacer arm to the commercial matrix, a suspension MP matrix (5 g) was prepared in toluene (60 mL), to which were added (3-chloropropyl)trimethoxysilane (5 mL) and triethylamine (0.5 mL), used as catalyst. After refluxing 24h and under magnetic stirring, the suspension was cooled down, the solid isolated by filtration and washed with toluene, ethanol/water (1:1), distilled water and, finally, with methanol. The matrix with the spacer is thus obtained and designated as MP-SilPrCl.

Subsequently, the matrix MP-SilPrCl was reacted with 1-methylimidazole in order to obtain the MP matrix functionalized with the IL 1-methylimidazolium chloride. To a suspension of matrix MP (5 g) with the spacer (MP-SilPrCl) in toluene (60 mL), 1-methylimidazole (5 mL) was added. This suspension was refluxed during 24 h under magnetic stirring (550 rpm). After this period, the solid was filtrated and washed with methanol, distilled water and methanol and dried at 60 ºC, thereby yielding the chromatographic matrix functionalized with the IL (MP-SilPrMImCl).

In what concerns to the ¹³C CPMAS NMR-CPMAS analysis, specific settings were used in order to maximize the signal-noise ratio: v₁¹³ C = 55 kHz; *recycle delay,* 4 s; contact period, 1-2 ms; NS = 1k; vR = 12 kHz. The ¹³C CPMAS spectra of matrices MP and MP-SilPrMImCl are represented in **Fig. 2****,** and the presence of peaks with chemical shifts within the intervals 120-130 and 35-40 ppm was observed in the MP-SilPrMImCl matrix spectrum, which are not present in matrix MP. If on one hand, the signal with chemical shift at 37 ppm is due to the methyl group present as alkyl chain of the imidazolium, the signals at 120-130 ppm are characteristic of the aromatic carbon atoms, which are solely present in the imidazolium aromatic ring. In addition, the elemental analysis of MP and MP-SilPrMImCL matrices was also performed to quantitatively determine the content in carbon, hydrogen and nitrogen present therein, according to **Fig. 3****.** In general, nitrogen was detected in matrix MP-SilPrMImCl, which is not present in the commercial macroporous matrix, confirming the results obtained by ¹³C CPMAS on the correct functionalization of the prepared matrix. The superficial charge of matrices MP and MP-SilPrMImCl was also evaluated by determining the PZC at different pH values. As represented in **Fig. 4****,** it was verified that the pH at which the matrices present a null PZC was, respectively, 3.7 and 8.7, for matrices MP and MP-SilPrMImCl, this change being due to the presence of IL 1-methylimidazolium chloride. Finally, by scanning electron microscopy, **Fig. 5****,** it was verified that the functionalization of matrix MP with the IL did not induce significant alterations on its morphology.

The nucleic acids sample comprises RNA, preferably ImwRNA, and gDNA and derives from an *Escherichia coli* DH5α fermentative process. To this purpose, the bacterial cellular growth was performed in a suitable culture medium (12g/L tryptone, 24g/L yeast extract, 4mL/L glycerol, 0.017M KH₂PO₄, 0.072M K₂HPO₄) under specific conditions of orbital rotation (250 rpm), and temperature (37 °C), in particular during 7 hours. Subsequently, bacterial lysis was performed employing the lysis solution that contains the guanidinium thiocyanate salt, followed by a concentration step with isopropanol and a washing step with ethanol. Finally, the sample was solubilized with 0.05 % diethyl pyrocarbonate (DEPC)-treated bidistilled water, this constituting the nucleic acids sample that contains RNA, preferably ImwRNA, and gDNA, lately employed in the separation assays. According to example 1, the matrix MP-SilPrMImCl can be used in conditions that mainly favor electrostatic or hydrophobic interactions, due to its multimodal character. In fact, according to the structure of the chromatographic ligand used, the IL 1-methylimidazolium chloride, it is verified that this exhibits an aromatic ring, which: 1) shows high hydrophobicity, making possible the establishment of hydrophobic interactions; 2) likewise presents a positive charge center, allowing the establishment of electrostatic interactions with negatively charged biomolecules, acting hereby as anion exchanger with the counter-ion chloride initially present. However, in order to essentially promote hydrophobic interactions, high concentrations of (NH₄)₂SO₄ are used and, considering the high eutrophication potential of (NH₄)₂SO₄, along with the fact that it is obtained from non-renewable sources, leads to the fact that the use of high concentrations of this reagent greatly increases the environmental footprint of a chromatographic process. This way, the ability of the matrix for establishing predominantly electrostatic interactions of the matrix MP-SilPrMImCl was explored, aiming the separation of nucleic acids. Initially, a 10 mm diameter and 26 mm height chromatographic column (Ge Healthcare, Uppsala, Sweden) was packed with 2 mL of hydrated matrix MP-SilPrMImCl, and the operation temperature in the column was set at 25 °C, controlled with a thermostatized bath; chromatographic assays were performed in the ÄKTA Avant system under the control of *software* Unicorn 6 (Ge Healthcare, Uppsala, Sweden). After testing different stepwise gradients with distinct ionic strengths, the optimized strategy consisted in the equilibrium of MP-SilPrMImCl matrix with 0.380 M NaCl (in 10 mM Tris-HCI, pH 8), followed by the increase in the concentration of NaCl to 1 M, thus obtaining 2 chromatographic peaks, which are represented in **Fig. 7-A** by 1 and 2, respectively. The samples represented by 1 and 2 were separately collected, concentrated and desalted using vivaspin concentrators (5000 MWCO, Sartorius, Gottingen, Germany), and were finally analyzed in a 1 % (m/v) agarose gel, containing GreenSafe Premium (Nzytech, Lisbon, Portugal) at a concentration of 0.014 µL/mL of agarose gel. The analysis by agarose gel electrophoresis of peaks 1 and 2 is shown in **Fig. 7B****,** which includes the fraction S, the initial sample wherein two bands are observed, one with higher molecular weight and the other with lower molecular weight which correspond, respectively, to gDNA and RNA, preferably ImwRNA. In what concerns to the chromatographic peaks, in peak 1 solely RNA, preferably ImwRNA, elutes, with a lower ionic strength, whereas in peak 2 it elutes gDNA. This process typically corresponds to a negative chromatographic process, since the biomolecule of interest is recollected in the binding step. Indeed, this strategy has two major advantages: the RNA, preferably ImwRNA, is recovered with a lower salt concentration, showing a lower residence time in the system, factors that contribute to maintain the integrity, stability and biological activity of RNA.

The regeneration of chromatographic matrices represents, in general terms, a way to remove components that remain adsorbed to the matrix even after the elution steps. In this case in particular it assumes special relevance once it is required to reestablish the counter-ion of the immobilized chromatographic ligand - the chloride ion. Even after several usage cycles, the matrix MP-SilPrMImCl maintains, in general, its atomic composition **(****Fig. 3****),** as well as it maintains its reproducibility **(****Fig. 9****).** In fact, although non-specific adsorption of RNA biomolecules, or contacts among the RNA molecules are observed as the number of chromatographic assays performed increases, the purity of the RNA, preferably ImwRNA, recovered in peak 1, however, is not compromised. Finally, the dynamic binding capacity for RNA, preferably ImwRNA, was also determined at 10 and 50 % of the maximum capacity of matrix MP-SilPrMImCl, and the following values were obtained, 3.68 and 12.04 mg/mL. In short, the strategy reported for the separation of tRNA from gDNA can assume a special relevance in the preparation of RNA for therapeutic and diagnostic applications or for molecular biology general applications.

### Application example 3:

In an embodiment, the separation of RNA, preferably ImwRNA, from gDNA by multimodal chromatography, using the matrix MP-SilPrMImCl was performed as described below.

In an embodiment, the chromatographic matrix described in this disclosure was obtained from the commercial macroporous matrix Toyopearl® AF-Epoxy-650M, in which a spacer arm was immobilized in a first instance, and then 1-methylimidazole was bound to the spacer arm, thereby leading to the formation of the chromatographic ligand. Initially, envisaging the covalent bond of the spacer arm to the commercial matrix, a suspension of MP matrix (5g) was prepared in toluene (60 mL), to which were added (3-chloropropyl)trimethoxysilane (5 mL) and triethylamine (0.5 mL), used as catalyst. After refluxing 24h and under magnetic stirring, the suspension was cooled down, the solid isolated by filtration and washed with toluene, ethanol/water (1:1), distilled water and, finally, with methanol. The matrix with the spacer is thus obtained and designated as MP-SilPrCl.

Subsequently, the matrix MP-SilPrCl was reacted with 1-methylimidazole aiming to obtain the MP functionalized with the IL 1-methylimidazolium chloride. To a suspension of matrix MP with the spacer (MP-SilPrCl) (5 g) in toluene (60 mL), 1-methylimidazole (5 mL) was added. This suspension was refluxed during 24 h under magnetic stirring (550 rpm). After this period, the solid was filtrated and washed with methanol, distilled water and methanol and dried at 60 ºC, thereby yielding the chromatographic matrix functionalized with the IL (MP-SilPrMImCl).

In what concerns to the ¹³C CPMAS NMR-CPMAS analysis, the following specific settings were used in order to maximize the signal-noise ratio: *v*₁¹³ C = 55 kHz; *recycle delay,* 4 s; contact period, 1-2 ms; NS = 1k; vR = 12 kHz. The ¹³C CPMAS spectra of matrices MP and MP-SilPrMImCl are represented in **Fig. 2****,** and the presence of peaks with chemical shifts within the intervals 120-130 and 35-40 ppm was observed in the MP-SilPrMImCl matrix spectrum, which are not present in matrix MP. If on one hand, the signal with chemical shift at 37 ppm is due to the methyl group present as alkyl chain of the imidazolium, the signals at 120-130 ppm are characteristic of the aromatic carbon atoms, which are solely present in the imidazolium aromatic ring. In addition, the elemental analysis of MP and MP-SilPrMImCL matrices was also performed to quantitatively determine the content in carbon, hydrogen and nitrogen therein, according to **Fig. 3****.** In general, nitrogen was detected in matrix MP-SilPrMImCl, which is not present in the commercial macroporous matrix, confirming the results obtained by ¹³C CPMAS on the correct functionalization of the prepared matrix. The superficial charge of matrices MP and MP-SilPrMImCl was also evaluated by determining the PZC at different pH values. As represented in **Fig. 4****,** it was verified that the pH at which the matrices present a null PZC was, respectively, 3.7 and 8.7, for matrices MP and MP-SilPrMImCl, this change being due to the presence of IL 1-methylimidazolium chloride. Finally, by scanning electron microscopy, **Fig. 5****,** it was verified that the functionalization of matrix MP with the IL did not induce significant alterations on its morphology.

The nucleic acids sample comprises RNA, preferably ImwRNA, rRNA and gDNA and derives from an *Escherichia coli* DH5α fermentative process. To this purpose, the bacterial cellular growth was performed in a suitable culture medium (12 g/L tryptone, 24 g/L yeast extract, 4 mL/L glycerol, 0.017 M KH₂PO₄, 0.072 M K₂HPO₄) under specific conditions of orbital rotation (250 rpm), and temperature (37 °C), in particular during 2 hours. Subsequently, bacterial lysis was performed employing the lysis solution that contains the guanidinium thiocyanate salt, followed by a concentration step with isopropanol and a washing step with ethanol. Finally, the sample was solubilized with 0.05 % diethyl pyrocarbonate (DEPC)-treated bidistilled water, this constituting the nucleic acids sample that contains RNA, preferably ImwRNA and rRNA, and gDNA, lately employed in the separation assays.

According the example 1, the matrix MP-SilPrMImCl can be used in conditions that mainly favor electrostatic or hydrophobic interactions, due to its multimodal character. In fact, according to the structure of the chromatographic ligand used, the IL 1-methylimidazolium chloride, it is verified that it exhibits an aromatic ring, which: 1) shows high hydrophobicity, making possible the establishment of hydrophobic interactions; 2) likewise presents a positive charge center, allowing the establishment of electrostatic interactions with negatively charged biomolecules, acting hereby as anion exchanger with the counter-ion chloride initially present. However, to mainly promote hydrophobic interactions, high concentrations of (NH₄)₂SO₄ are used, and considering the high eutrophication potential of (NH₄)₂SO₄, along with the fact that it is obtained from non-renewable sources, leads to the fact that the use of high concentrations of this reagent greatly increases the environmental footprint of a chromatographic process. This way, the ability of the matrix for establishing predominantly electrostatic interactions of matrix MP-SilPrMImCl was explored, aiming the separation of nucleic acids. Initially, a 10 mm diameter and 26 mm height chromatographic column (Ge Healthcare, Uppsala, Sweden) was packed with 2 mL of hydrated matrix MP-SilPrMImCl, and the operation temperature in the column was set at 25 °C, controlled with a thermostatized bath; the chromatographic assays were performed in ÄKTA Avant system under the control of *software* Unicorn 6 (Ge Healthcare, Uppsala, Sweden), which allows not only an automated collection of the different fractions, but also allows the on-line monitoring of diverse parameters such as conductivity, absorbance and pH. After being tested different stepwise gradients with distinct ionic strengths, the optimized strategy consisted in the equilibrium of MP-SilPrMImCl matrix with 0.380 M NaCl (in 10 mM Tris-HCI, pH 8), followed by the increase in the concentration of NaCl to 0.41 M and 0.5 M (in 10 mM Tris-HCI, pH 8) by two stepwise gradients and 3 chromatographic peaks were obtained, which are represented in **Fig. 8-A** by 1, 2 and 3, respectively. The samples represented by 1, 2 and 3 were separately collected, concentrated and desalted using vivaspin concentrators (5000 MWCO, Sartorius, Gottingen, Germany), and were finally analyzed in a 1 % (m/v) agarose gel, containing GreenSafe Premium (Nzytech, Lisbon, Portugal) at a concentration of 0.014 µL/mL of agarose gel.

The results are shown in **Fig. 8B****,** and it is possible to observe 4 bands in the initial sample - Fraction S -, which correspond to distinct species of nucleic acids, namely, the band with higher molecular weight corresponds to gDNA, the two bands with intermediate molecular weights correspond to rRNA and the band with a lower molecular weight corresponds to ImwRNA. On the other hand, it was verified that ImwRNA elutes in peak 1, gDNA in peak 2 and rRNA in peak 3, thereby confirming that the chromatographic strategy implemented and developed with MP-SilPrMImCl matrix allows an effective separation of ImwRNA, rRNA and gDNA, coming from a bacterial extract.

Although in the present disclosure only particular embodiments thereof have been represented and described, the person skilled in the art will know how to introduce modifications and replace some technical features with other equivalent ones, depending on the requirements of each situation, without departing from the scope of protection defined by the appended claims.

The embodiments presented are combinable with each other.

The following claims further define embodiments.

### References:

[1] R. Martins, J.A. Queiroz, F. Sousa, Ribonucleic acid purification, J Chromatogr A (2014), 1355, 1-14.
[2] P. Pereira, J.A. Queiroz, A. Figueiras, F. Sousa, Affinity approaches in RNAi-based therapeutics purification, J Chromatogr B Analyt Technol Biomed Life Sci (2016), 1021, 45-56.
[3] M.G. Freire, A.F.M. Cláudio, J.M.M. Araújo, J.A.P. Coutinho, I.M. Marrucho, J.N.C. Lopes, L.P.N. Rebelo, Aqueous biphasic systems: a boost brought by using ionic liquids, Chem Soc Rev (2012), 41, 4966-4995.
[4] L. Brown, M.J. Earle, M.A. Gîlea, N.V. Plechkova, K.R. Seddon, Ionic liquid-chromatography: a new general purpose separation methodology, Top Curr Chem (Cham) (2017), 375 (5), 74.
[5] X. Shi, L. Qiao, G. Xu, Recent development of ionic liquid stationary phases for liquid chromatography, J Chromatogr A (2015), 1420, 1-15.

## Claims

1. Chromatographic matrix that comprises:
a porous solid support,
a spacer covalently bound to the porous solid support,
a multimodal ligand covalently bound to the spacer, and
wherein the multimodal ligand is able to bind a nucleic acid.

2. Matrix according to the previous claim, wherein the density of the multimodal ligand in the porous solid support ranges between 0.01 - 2 mmol of multimodal ligand/g of porous solid support.

3. Matrix according to any one of the previous claims, wherein the density of the multimodal ligand in the porous solid support ranges between 0.5 and 1 mmol of multimodal ligand/g of porous solid support.

4. Matrix according to any one of the previous claims, wherein the density of the multimodal ligand in the porous solid support is 0.726 mmol of multimodal ligand/g of porous solid support.

5. Matrix according to any one of the previous claims, wherein the molar ratio multimodal ligand:spacer is 1:1.

6. Matrix according to any one of the previous claims, wherein the multimodal ligand is an ionic liquid.

7. Matrix according to the previous claim, wherein the ionic liquid is selected from a family of imidazolium cations, pyridinium cations, piperidinium cations, quaternary ammonium cations or quaternary phosphonium cations, preferably combined with organic and inorganic anions.

8. Matrix according to any one of the previous claims 6-7, wherein the ionic liquid is selected from a family of imidazolium cations combined with an inorganic anion.

9. Matrix according to any one of the previous claims 6-8, wherein the ionic liquid is 1-methylimidazolium chloride.

10. Matrix according to any one of the previous claims, wherein the spacer is selected from the following group: (3-chloropropyl)trimethoxysilane, (3-chloropropyl)triethoxysilane, (3-aminopropyl)trimethoxysilane, (3-mercaptopropyl)trimethoxysilane, (N-N-dimethylaminopropyl)trimethoxysilane or [3-(2-aminoethylamino)propyl]trimethoxysilane.

11. Matrix according to any one of the previous claims, wherein the spacer is (3-chloropropyl)trimethoxysilane.

12. Matrix according to any one of the previous claims, wherein the spacer is (3-chloropropyl)trimethoxysilane and the ionic liquid is 1-methylimidazolium chloride.

13. Matrix according to any one of the previous claims, wherein the porous solid support is a polymer, preferably a polymer functionalized with epoxy groups, still more preferred a methacrylic polymer functionalized with epoxy groups.

14. Matrix according to the previous claim, wherein the epoxylated methacrylic polymer has a particle size between 40 and 90 µm, and a mean pore size of 1000 Å.

15. Matrix according to any one of the previous claims, wherein the nucleic acid is RNA and/or genomic DNA, preferably wherein the RNA comprises low molecular weight RNA and/or ribosomal RNA.

16. Kit for the separation or diagnostic of nucleic acids that comprises the chromatographic matrix described in any one of the previous claims.

17. Method for isolating nucleic acids from a sample, comprising the following steps:
contacting the sample with the chromatographic matrix described in any one of the previous claims 1-15;
separately eluting nucleic acids from the sample.

18. Method according to the previous claim, wherein the sample is a bacterial lysate sample comprising RNA and genomic DNA, preferably wherein the RNA comprises low molecular weight RNA and/or ribosomal RNA.

19. Method according to any one of the previous claims 17-18, wherein the step of separately eluting the nucleic acids comprises separately eluting low molecular weight RNA and genomic DNA, preferably wherein the step of separately eluting nucleic acids comprises separately eluting low molecular weight RNA and/or ribosomal RNA and/or genomic DNA.

20. Method according to any one of the previous claims 17-19, wherein the step of separately eluting the nucleic acids from the sample is performed with an increase in the ionic strength using a buffer with 10 mM Tris-HCI pH 8 and 1 M NaCl and following a stepwise gradient.

21. Method according to any one of the previous claims 17-20, wherein the step of separately eluting the nucleic acids from the sample comprises eluting RNA, preferably ImwRNA, with 10 mM Tris-HCI pH=8 and 0.3 M NaCl - 0.4 M NaCl, preferably 0.38 M NaCl.

22. Method according to any one of the previous claims 17-21, wherein the step of separately eluting the nucleic acids from the sample comprises eluting genomic DNA with 10 mM Tris-HCI pH=8 and 0.5 M NaCl - 1 M NaCl, preferably 1 M NaCl.

23. Method according to any one of the previous claims 17-22, wherein the step of separately eluting the nucleic acids from the sample comprises eluting ribosomal RNA with 10 mM Tris-HCI pH=8 and 0.46 M NaCl - 1 M NaCl, preferably 0.5 M NaCl.

24. Method according to any one of the previous claims 17-23, wherein the step of separately eluting the nucleic acids from the sample is performed by a decrease in the concentration of 2 M (NH₄)₂SO₄ in 10 mM Tris-HCI, pH 8, following a stepwise gradient.

25. Method for preparing the chromatographic matrix according to any one of the previous claims 1-15, comprising the following steps:
covalently binding a spacer to a porous solid support;
covalently binding a multimodal ligand to the spacer from the previous step.

26. Method according to the previous claim wherein the multimodal ligand is an ionic liquid selected from a family of imidazolium cations, pyridinium cations, piperidinium cations, quaternary ammonium cations or quaternary phosphonium cations, preferably combined with organic and inorganic anions, preferably the ionic liquid is selected from a family of imidazolium cations combined with an inorganic anion, particularly the ionic liquid is 1-methylimidazolium chloride.

27. Method according to any one of the previous claims 25-26, wherein the spacer is selected from the following group: (3-chloropropyl)trimethoxysilane, (3-chloropropyl)triethoxysilane, (3-aminopropyl)trimethoxysilane, (3-mercaptopropyl)trimethoxysilane, (N-N-dimethylaminopropyl)trimethoxysilane or [3-(2-aminoethylamino)propyl]trimethoxysilane, particularly the spacer is (3-chloropropyl)trimethoxysilane.

28. Method according to any one of claims 25-27, wherein the spacer is (3-chloropropyl)trimethoxysilane and the ionic liquid is 1-methylimidazolium chloride.

29. Method according to any one of the previous claims 25-28, wherein the porous solid support is a polymer, preferably a polymer functionalized with epoxy groups, still more preferred a methacrylic polymer functionalized epoxy groups wherein the epoxylated methacrylic polymer has a particle size between 40 and 90 µm, and a mean pore size of 1000 Å.

30. Use of the chromatographic matrix described in any one of claims 1-15 in a chromatographic process for isolating and/or purifying and/or separating nucleic acids, preferably for separating low molecular weight RNA from genomic DNA, still more preferred for separating low molecular weight RNA, from ribosomal RNA, from genomic DNA.
